(19) Europäisches Patentamt
European Patent Office
Office européen des brevets

(11) **EP 3 738 925 B1**

(12) **EUROPEAN PATENT SPECIFICATION**

(45) Date of publication and mention
of the grant of the patent:
**13.09.2023  Bulletin 2023/37**

(21) Application number: **18902336.9**

(22) Date of filing: **26.09.2018**

(51) International Patent Classification (IPC):
*C01B 11/02* (2006.01)   *A61L 2/22* (2006.01)
*A61L 9/01* (2006.01)   *A61L 9/14* (2006.01)
*B05B 9/04* (2006.01)   *B65D 83/14* (2006.01)
*B65D 83/68* (2006.01)   *C09K 3/30* (2006.01)
*A61L 101/06* (2006.01)

(52) Cooperative Patent Classification (CPC):
**C01B 11/024; A61L 2/22; A61L 9/01; A61L 9/14;
B65D 83/62; B65D 83/682; C09K 3/30;**
A61L 2202/15; A61L 2209/134

(86) International application number:
**PCT/JP2018/035568**

(87) International publication number:
**WO 2019/146158 (01.08.2019 Gazette 2019/31)**

(54) **CHLORINE DIOXIDE GENERATING AEROSOL**

CHLORDIOXID ERZEUGENDES AEROSOL

AÉROSOL GÉNÉRANT DU DIOXYDE DE CHLORE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **29.01.2018   JP 2018012305**

(43) Date of publication of application:
**18.11.2020   Bulletin 2020/47**

(73) Proprietor: **Toyo Aerosol Industry Co., Ltd.
Tokyo 141-0022 (JP)**

(72) Inventors:
• **NAKAJIMA, Yasutomo
Tokyo 141-0022 (JP)**

• **TERASHIMA, Remi
Tokyo 141-0022 (JP)**

(74) Representative: **Vigand, Philippe et al
Novagraaf International SA
Chemin de l'Echo 3
1213 Onex - Genève (CH)**

(56) References cited:
**EP-A1- 2 886 625        JP-A- 2000 185 908
JP-A- 2000 239 003       JP-A- 2004 143 004
JP-A- 2004 337 582       JP-A- 2008 094 662
US-A1- 2005 079 123      US-A1- 2010 036 305
US-A1- 2013 202 484      US-B2- 8 840 847**

**Description**

[Technical Field]

The present invention relates to an aerosol product that generates chlorine dioxide.

[Background Art]

Chlorine dioxide $ClO_2$, which exhibits strong oxidizing power, is used as a disinfectant, virus remover or freshener. Chlorine dioxide is generally used in the form of aqueous solution, from the viewpoint of handleability, but such solutions are problematic in terms of stability. Accordingly, chlorine dioxide is used in the state of a chlorite salt such as sodium chlorite, and in the form of so-called stabilized chlorine dioxide aqueous solutions having the pH adjusted to alkaline pH.

As a means for eliciting continuous release of chlorine dioxide gas at a given concentration using such stabilized chlorine dioxide aqueous solutions, a chlorine dioxide solution agent that includes a dissolved chlorine dioxide gas, a chlorite salt and a pH adjusting agent, as constituent components, has been proposed (for instance PTL 1).

[Citation List]

[Patent Literature]

[PTL 1] Japanese Patent No. 3110724

[Summary of Invention]

[Technical Problem]

The chlorine dioxide solution agent of PTL 1 allows continuously releasing chlorine dioxide gas of a given concentration for a certain time. Studies by the inventors have however revealed that such a chlorine dioxide solution agent is insufficient in terms of generating highly fast-acting chlorine dioxide.

Another conceivable means, from the viewpoint of fast-acting properties, involves preparing separately a chlorite salt such as sodium chlorite and an activator such as citric acid for activating the chlorite salt, to thereby generate chlorine dioxide, and then mixing the chlorite salt and the activator prior to use. Once the foregoing are mixed, however, fast-acting properties cease to be obtained with the passage of time. JP 2004 337582 A, US 2013/202484 A1, US 2005/079123 A1, US 8 840 847 B2 and EP 2 886 625 A1 disclose aerosol devices to generate chlorine dioxide.

It is an object of the present invention to solve such a problem. Specifically, it is an object of the present invention to provide an aerosol product capable of continuously generating highly fast-acting chlorine dioxide.

[Solution to Problem]

The present invention relates to an aerosol product as defined in claim 1.

[Advantageous Effects of Invention]

The present invention succeeds in providing an aerosol product capable of generating highly fast-acting chlorine dioxide continuously.

[Brief Description of Drawings]

[Fig. 1] Fig. 1 is a schematic diagram illustrating an example of an aerosol product of a double structure container.
[Fig. 2] Fig. 2 is a schematic diagram illustrating an example of an actuator of a double structure container.
[Fig. 3] Fig. 3 is a schematic diagram of an aerosol product of double can type.
[Fig. 4] Fig. 4 is an arrangement example of a petri dish in a virus titer comparison test.

[Description of Embodiments]

When the aqueous solution of a chlorite salt and the aqueous solution of an acidic substance are mixed, the aqueous solution of a chlorite salt is activated, and chlorine dioxide is generated. In the present invention, thus, chlorine dioxide starts being generated immediately after discharge, and accordingly chlorine dioxide can be generated from a target discharge portion, with a very fast action (in a state of fast generation rate). For instance, virus titers can be reduced within 1 hour after discharge.

By virtue of these pronounced fast-acting properties, the target discharge portion is affected less readily by organic matter or the like than conventional chlorine dioxide solution agents that utilize a stabilized chlorine dioxide aqueous solution.

At times other than discharge, the first liquid containing the aqueous solution of a chlorite salt and the second liquid containing the aqueous solution of an acidic substance are respectively filled into the first container and the second container, within the aerosol product, and hence can be present in a stable state. Accordingly, highly fast-acting chlorine dioxide can be generated continuously.

Materials that can be used in the present invention will be explained next.

<Aqueous solution of chlorite salt>

An aqueous solution of a chlorite salt is used as the first liquid of the present invention. Preferably, the chlorite salt includes at least one selected from the group consisting of sodium chlorite, potassium chlorite, lithium chlorite, calcium chlorite, magnesium chlorite and barium chlorite. Sodium chlorite, potassium chlorite and lithium chlorite are more preferable, and sodium chlorite even yet more preferable. The aqueous solution of sodium chlorite that is used may be a commercially available one, for instance Purogene (by Bio-Cide Japan Co., Ltd.).

A buffering agent or the like may be added as needed to the aqueous solution of a chlorite salt.

The concentration of the aqueous solution of a chlorite salt in the aerosol product depends on the concentration of the aqueous solution of an acidic substance that is used at the same time, and accordingly is not particularly limited, but is preferably 0.1 to 100,000 ppm, more preferably 1 to 100,000 ppm, and yet more preferably 20 to 10,000 ppm, on a mass basis.

The pH of the aqueous solution of a chlorite salt depends for instance on the chlorite salt that is used, and the buffering agent that is utilized as needed, and accordingly is not particularly limited, but from the viewpoint of stability is preferably from 5 to 12.5, more preferably from 5 to 12, yet more preferably 5 or higher and less than 9, and particularly preferably 5 or higher and less than 8. In a case where the aqueous solution of a chlorite salt contains a buffering agent, for instance pH is preferably from 5 to 12, more preferably 5 or higher and less than 9, yet more preferably from 5 to 8.5, and particularly preferably 5 or higher and less than 8.

<Aqueous solution of acidic substance>

In the present invention an aqueous solution of an acidic substance is citric acid.

The concentration of the aqueous solution of an acidic substance is 1 to 20 mass%.

In the present invention, the first liquid is an aqueous solution of sodium chlorite, and the second liquid is an aqueous solution of citric acid. Chlorine dioxide can be generated for instance in accordance with the reaction below, through mixing of sodium chlorite and citric acid.

$$5NaClO_2 + 4C_5H_7O_5(COOH) \rightarrow 4ClO_2 + 2H_2O + 5Na^+ + C_5H_7O_5COO^-$$

<Other additives>

Other additives may be used in the aerosol product of the present invention, so long as the effect of the invention is not impaired thereby. For instance known surfactants, thickeners, fragrances and so forth can be added. Preferably the additives are contained in the second liquid, from the viewpoint of stability. In the present invention the containers filled with the first liquid and the second liquid are separate, and accordingly the effect of the additives can be imparted to the first liquid and the second liquid, while the stability of the chlorite salt which is the first liquid is maintained.

<Propellant>

The propellant is not particularly limited, and a liquefied gas or a compressed gas may be used. Preferred herein is a compressed gas, for instance nitrous oxide gas, nitrogen gas, carbon dioxide gas, or a mixed gas of the foregoing.

<Aerosol product>

The aerosol product of the present invention includes a first container filled with a first liquid and a second container filled with a second liquid. For instance container forms such as a double-structure container or a double can type can be used so long as the first liquid and the second liquid can be filled separately into the container, and the foregoing can be discharged and mixed.

A double-structure container includes for instance an outer container provided with a propellant filling space filled with a propellant, a first container, and a second container, and a discharge mechanism for discharging and mixing a first liquid and a second liquid from the first container and the second container, wherein the propellant filling space is formed between the first container, the second container and the outer container.

The double-structure container includes the outer container provided with the propellant filling space filled with a propellant, the first container filled with the first liquid, and the second container filled with the second liquid. The first liquid and the second liquid can be discharged and mixed simultaneously, from the first container and the second container, on account of the pressure of the propellant with which the propellant filling space is filled.

An example of a double-structure container having a discharge mechanism will be explained next with reference to accompanying drawings.

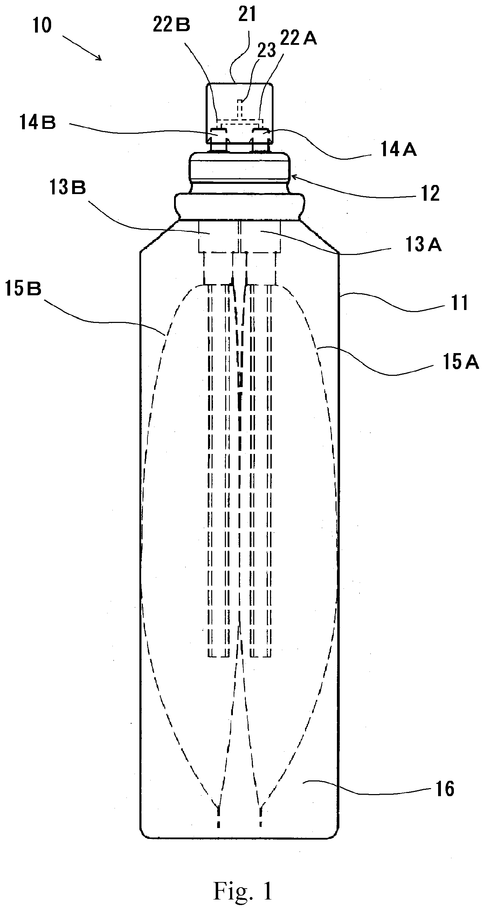

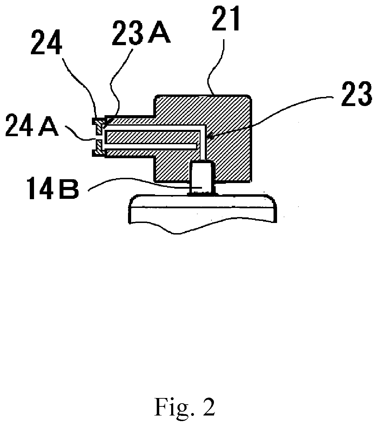

Fig. 1 is a schematic diagram illustrating an example of an aerosol product of a double-structure container, and Fig. 2 is a schematic diagram illustrating a cross section of an actuator (discharge mechanism) of the double-structure container of Fig. 1.

The double-structure container 10 has a metal-made pressure container (outer container) 11 provided with an aerosol valve 12. In the interior of the of the pressure container (outer container) 11 there are provided a first inner bag 15A that demarcates a first container for filling of a first liquid, and a second inner bag 15B that demarcates a second container for filling of a second liquid. A propellant filling space 16 for filling of a propellant is formed by respective gaps between the pressure container (outer container) 11, and the first inner bag 15A and the second inner bag 15B.

A first stem 14A and a second stem 14B each having a stem in the interior thereof are provided in the aerosol valve 12, so that the first stem 14A and the second stem 14B can slide vertically within a first housing 13A and a second housing 13B, respectively. A shared actuator (discharge mechanism) 21 is provided at the upper end of the first stem 14A and the second stem 14B.

In Fig. 1 the constituent elements positioned within the pressure container 11 and the actuator 21 are depicted with dashed lines.

In the aerosol product of the present invention, a discharge mixture is discharged in the form of a mist. The structure of the actuator for discharge in a mist form is not particularly limited, and a known structure can be used herein. Examples thereof include forms such as those below.

The shared actuator 21 is provided with a first actuator passage 22A that communicates with the stem passage of the first stem 14A, a second actuator passage 22B that communicates with the stem passage of the second stem 14B, and an L-shaped mixing space 23 one end of which communicates with the first actuator passage 22A and the second actuator passage 22B, the other end communicating with a discharge port 24A of a nozzle forming member 24. The mixing space 23 is provided with a swirl passage 23A having a function of dividing the first liquid and the second liquid into fine solution droplets as the first liquid and the second liquid pass through the swirl passage 23A.

By using such a discharge mechanism it becomes possible to discharge and mix, in the form of a mist, the first liquid and the second liquid from the first inner bag 15A and the second inner bag 15B, on account of the pressure of the propellant that fills the propellant filling space.

In Fig. 1 an embodiment in which two types of content liquid, namely the first liquid and the second liquid, are discharged and mixed has been explained, but a double-structure container in which three or more types of content liquid are discharged and mixed may be adopted. Specifically three or more types of content liquid can be discharged and mixed by providing for instance a third inner bag, a third housing and a third stem within the pressure container 11. For instance, the above other additive or the like can be discharged and mixed by providing a third inner bag and a fourth inner bag. Various solutions can be stored stably as a result.

In Fig. 1 the inner bags are arrayed parallelly to each other, but the arrangement of the inner bags may adopt various known forms. The first inner bag and the second inner bag may be arrayed vertically. For instance, the first inner bag may be arrayed at the top and the second inner bag at the bottom, with the actuator, the first inner bag and the second inner bag connected to each other by way of a tube or the like. The tube connecting the second inner bag and the actuator may be led around the exterior of the first inner bag, or may pass through the interior of the first inner bag but so as not to come into contact with the first liquid.

In a double can-type form, two aerosol products are integrated with each other, and the first liquid and the second liquid are discharged and mixed simultaneously by a propellant.

For instance such a double can type includes a first inner container filled with the first liquid and a second inner container filled with the second liquid, a first outer container housing the first inner container, a second outer container housing the second inner container, and a discharge mechanism for discharging and mixing the first liquid and the second liquid, wherein spaces formed between the first inner container and the first outer container, and between the second inner container and the second outer container are filled with a propellant.

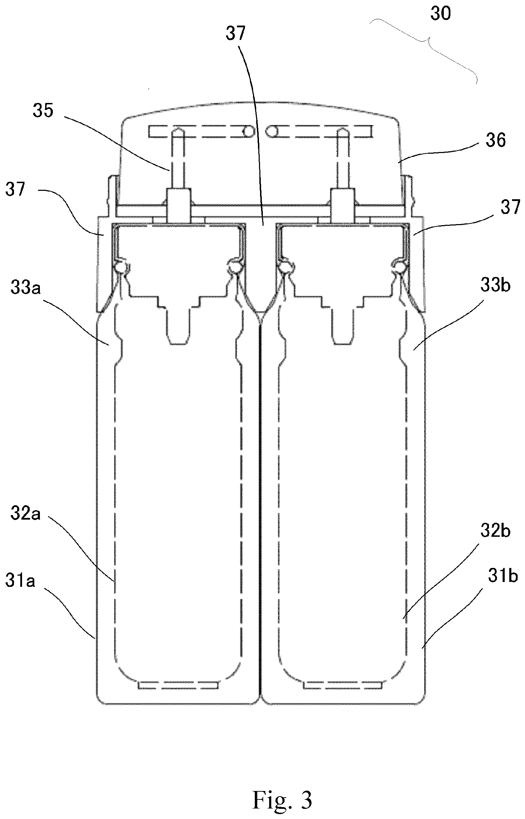

Fig. 3 is a schematic diagram of an aerosol product of double can type.

The aerosol product 30 is provided with a first outer container 31a, a second outer container 31b, and an actuator (discharge mechanism) 36 having an actuator passage 35. The first outer container 31a houses the first inner container 32a, and the first liquid is filled in the first inner container 32a. The second outer container 31b houses the second inner container 32b, and the second liquid is filled in the second inner container 32b. The first outer container 31a and the second outer container 31b are fixed by a fixed plate 37.

Within the aerosol product a filling space for first propellant 33a and a filling space for second propellant 33b are formed between the first outer container 31a and the first inner container 32a, and between the second outer container 31b and the second inner container 32b, respectively, and the propellant filling spaces are filled with a propellant for discharging the first liquid and the second liquid from the discharge mechanism 35.

In Fig. 3 the containers are arrayed parallelly to each other, but the arrangement of the containers may adopt various known forms. For instance the first outer container and the second outer container may be arrayed vertically. The first outer container may be arrayed at the top and the second outer container at the bottom, with the actuator, the first outer container and the second outer container connected to each other by way of a tube or the like.

The discharge mechanism 35 has discharge ports at which the compositions that fill the first inner container 32a and the second inner container 32b are discharged and mixed. The first liquid and the second liquid discharged from the discharge ports are preferably discharged in the form of a mist.

The double can-type aerosol product may be of a form in which three or more types of content liquid are discharged and mixed, similarly to a double-structure container. Specifically, three or more types of content liquid can be discharged and mixed, by providing for instance a third outer container, a third inner container and a third propellant filling space.

The actuator (discharge mechanism) in Fig. 3 has two discharge ports, such that the first liquid and the second liquid are discharged from separate discharge ports in the form of a mist, and are mixed. Mixing of the first liquid and the second liquid may thus take place after discharge from the actuator (discharge mechanism).

The structure of the actuator (discharge mechanism) in the two forms, namely a double-structure container and a double can type, is not particularly limited. For instance a structure may be adopted, as illustrated in Fig. 2, in which the first liquid and the second liquid are mixed within the actuator (discharge mechanism), and are thereafter discharged in mist form; alternatively, the (discharge mechanism) may have two discharge ports, such that first liquid and the second liquid are discharged from separate discharge ports in the form of a mist, and are mixed, as in Fig. 3. The former is more preferable from the viewpoint of reactivity of the first liquid and the second liquid.

In the aerosol product of the present invention a mixing ratio of the first liquid and the second liquid (mass of the first liquid : mass of the second liquid) discharged from the actuator (discharge mechanism) depends on the concentration of the first liquid and of the second liquid, and accordingly is not particularly limited, but preferably lies in the range of 0.8:1.2 to 1.2:0.8.

<Containers>

The containers (inner container) filled with the first liquid and the second liquid are not particularly limited. Containers made up of resins, metals or combinations thereof can be used herein.

The outer containers are not particularly limited, so long as they are capable of withstanding the pressure of the propellant, and for instance known metals can be used in the containers.

Examples

The present invention will be explained next in concrete terms with reference to examples. The present invention is however not limited to the examples below.

<Example 1: Production of aerosol product 1>

The below-described aqueous solutions were prepared as the first liquid and the second liquid, and were filled into the first container and the second container of a double-structure container such as the one illustrated in Fig. 1. The void formed between the outer container, the first container and the second container of the double-structure container was filled with nitrogen gas, to yield Aerosol product 1. A discharge mechanism was used, such as the one illustrated in Fig. 2, that allowed discharge of a mixture in mist form, with the discharge amounts of the first liquid and the second liquid being set to 1:1, on a mass basis. The concentration of chlorite ion after discharge was 500 ppm.

(First liquid)

- Aqueous solution of sodium chlorite (chlorite ion concentration 1000 ppm; pH 8.9)

Diluted Purogene (by Bio-Cide Japan Co., Ltd.) was used herein

(Second liquid)

- 20 mass% aqueous solution of citric acid

<Comparative example 1>

Cleverin spray (by Taiko Pharmaceutical Co., Ltd.), which is a commercially available chlorine dioxide formulation, was used herein.

<Comparative example 2>

A dilution (chlorite ion concentration 500 ppm; pH 8.7) of the same aqueous solution of sodium chlorite that was used in example 1 was filled into an aerosol container, which was used as a comparative aerosol container. Nitrogen gas is used as the propellant, and the discharge form is a mist form.

<Virus-coated petri dish>

A diluted solution of feline calicivirus (FCV-F9) in phosphate-buffered saline was coated onto a petri dish, with drying, to prepare a virus-coated petri dish.

<Virus titer comparison test>

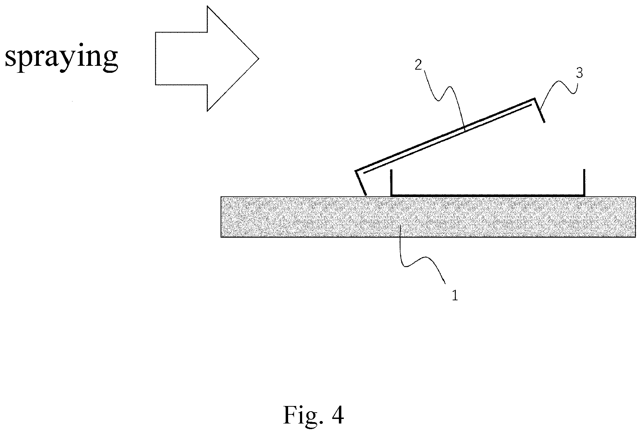

The effect of the chlorine dioxide agents of the example and comparative examples was assessed using the virus-coated petri dish prepared as described above. The product was sprayed in a garage 95 cm wide, 180 cm long and 160 cm high. A test stand 1 having a height of 60 cm was prepared in the garage, and a virus-coated petri dish 3 was disposed, as illustrated in Fig. 4, and the liquid was sprayed from a position at a height of 131 cm, so that the sprayed liquid did not come into direct contact with a virus-coated surface 2. The spray amount was set to be identical for each chlorine dioxide agent.

The petri dish was removed from the garage after 0 hours and after 1 hour from spraying, the virus was extracted, and the virus titer was evaluated. Each chlorine dioxide agent was evaluated after 0 hours and after 1 hour, and respective arithmetic mean values were calculated and evaluated. The results are given in Table 1.

Virus titers were evaluated in accordance with the $TCID_{50}$ method. The results of the examples are given with respect to 100 as the value in a case where distilled water is used as a spray sample.

The results in Table 1 indicate that the aerosol product of the present invention exhibits high fast-acting properties.

[Table 1]

|  | Example 1 | | Comparative example 1 | | Comparative example 2 | | Distilled water | |
|---|---|---|---|---|---|---|---|---|
| Elapsed time (h) | 0 | 1 | 0 | 1 | 0 | 1 | 0 | 1 |
| Comparison with D. W. | 100.00 | 8.25 | 100.00 | 21.50 | 100.00 | 26.10 | - | - |

In the table, the value "Comparison with D. W." refers to a numerical value with respect to 100 as the value of distilled water (D. W.)

<Titration test for effective chlorine dioxide>

The change over time in the effective chlorine dioxide concentration of each aqueous solution of sodium chlorite was checked in accordance with the method below.

- Measurement sample liquid 1 (no citric acid)

Herein Purogene (by Bio-Cide Japan Co., Ltd.) was diluted with purified water and was adjusted to 500 ppm, which is measurable with a BCI Titration Kit, to yield Measurement sample liquid 1.

- Measurement sample liquid 2 (activated with a 1 mass% aqueous solution of citric acid)

Herein Purogene (by Bio-Cide Japan Co., Ltd.) was diluted with purified water, to produce a 2000 ppm dilution of Purogene. Separately there was produced a 1 mass% aqueous solution of citric acid. Then 20 g each of the 2000 ppm dilution of Purogene and of the 1 mass% aqueous solution of citric acid were mixed, to produce an activated 1000 ppm dilution of Purogene. This dilution was further diluted two-fold, and was adjusted to 500 ppm, which is measurable by BCI Kit titration, to yield Measurement sample liquid 2.

- Measurement sample liquid 3 (activated with a 10 mass% aqueous solution of citric acid)

Herein Purogene (by Bio-Cide Japan Co., Ltd.) was diluted with purified water, to produce a 2000 ppm dilution of Purogene. Separately there was produced a 10 mass% aqueous solution of citric acid. Then 20 g each of the 2000 ppm dilution of Purogene and of the 10 mass% aqueous solution of citric acid were mixed, to produce an activated 1000 ppm dilution of Purogene. This dilution was further diluted two-fold, and was adjusted to 500 ppm, which is measurable with a BCI Titration Kit, to yield Measurement sample liquid 3.

- Measurement sample liquid 4 (activated with a 20 mass% aqueous solution of citric acid)

Herein Purogene (by Bio-Cide Japan Co., Ltd.) was diluted with purified water, to produce a 2000 ppm dilution of Purogene. Separately there was produced a 20 mass% aqueous solution of citric acid. Then 20 g each of the 2000 ppm dilution of Purogene and of the 20 mass% aqueous solution of citric acid were mixed, to produce an activated 1000 ppm dilution of Purogene. This dilution was further diluted two-fold, and was adjusted to 500 ppm, which is measurable with a BCI Titration Kit, to yield Measurement sample liquid 4.

o Titration procedure

Titration was carried out as follows using a BCI Titration Kit (by Bio-Cide Japan Co., Ltd.)

1. Addition of 10 drops of C/D Reagent #1 to a titration container.
2. Addition of 10 drops of C/D Reagent #2 to the container in 1.
3. Addition of 1 g of measurement sample liquid to the container in 2, with mixing.
4. Addition of 5 drops of C/D Reagent #3 to the container in 3.
5. Addition of one drop at a time of C/D Reagent #4 to the container in 4, until the solution becomes transparent.

Each measurement sample was titrated immediately following adjustment of the sample liquid (0 days), then after 3 hours (after 0.125 days), after 1 day, after 3 days, and after 35 days.

∘ Method for calculating effective chlorine dioxide

The effective chlorine dioxide concentration was calculated in accordance with the expression below, on the basis of the titration results. The results are given in Table 2.

$$\text{Effective chlorine dioxide (ppm)} = \text{number of drops} \times 5$$

The results in Table 2 indicated that if no citric acid was added, the effective chlorine dioxide of the aqueous solutions of sodium chlorite exhibited virtually no change, but decreased over time when citric acid was added. It is thus found that the aerosol product of the present invention can generate highly fast-acting chlorine dioxide stably, unaffected by the passage of time, as compared with a product of a type in which an activator such as citric acid and a chlorite salt are mixed.

[Table 2]

| Concentration (ppm) of effective chlorine dioxide | | | | |
|---|---|---|---|---|
| Time (days) | No citric acid | 1 mass% citric acid | 10 mass% citric acid | 20 mass% citric acid |
| 0 | 460 | 485 | 455 | 435 |
| 0.125 | 475 | 455 | 425 | 390 |
| 1 | 470 | 425 | 345 | 285 |
| 3 | 475 | 380 | 260 | 255 |
| 35 | 470 | 75 | 25 | 20 |

The change over time of the effective chlorine dioxide concentration when using hydrochloric acid or sulfuric acid was checked next. - Measurement sample liquid 5 (activated with a 1 mass% hydrochloric acid aqueous solution) - not according to the invention -.

Herein Purogene (by Bio-Cide Japan Co., Ltd.) was diluted with purified water, to produce a 2000 ppm dilution of Purogene. Separately there was produced a 1 mass% aqueous solution of hydrochloric acid. Then 20 g each of the 2000 ppm dilution of Purogene and of the 1 mass% aqueous solution of hydrochloric acid were mixed, to produce an activated 1000 ppm dilution of Purogene. This dilution was further diluted two-fold, and was adjusted to 500 ppm, which is measurable by BCI Kit titration, to yield Measurement sample liquid 5.

- Measurement sample liquid 6 (activated with 1 mass% sulfuric acid aqueous solution) - not according to the invention -.

Herein Purogene was diluted with purified water, to produce a 2000 ppm dilution of Purogene. Separately there was produced a 1 mass% aqueous solution of sulfuric acid. Then 20 g each of the 2000 ppm dilution of Purogene and of the 1 mass% aqueous solution of sulfuric acid were mixed, to produce an activated Purogene 1000 ppm dilution of Purogene. This dilution was further diluted two-fold, and was adjusted to 500 ppm, which is measurable by BCI Kit titration, to yield Measurement sample liquid 6.

- Measurement sample liquid 2 (activated with 1 mass% aqueous solution of citric acid)

Measurement sample liquid 2 produced in the same way as above was used herein.

The above measurement sample was titrated immediately following adjustment of the sample (0 days), then after 3 hours (after 0.125 days), after 1 day, after 3 days, and after 35 days. Sample solutions 5 and 6 were also titrated after 9 days and after 28 days. Titration was carried out using a BCI Titration Kit (by Bio-Cide Japan Co., Ltd.), in the same way as above, and the effective chlorine dioxide concentration ppm was calculated. The results are given in Table 3.

The results below reveal that the active component is deactivated quickly when a strong acid such as hydrochloric acid or sulfuric acid is used as an activator. The results reveal that the aerosol product of the present invention can generate highly fast-acting chlorine dioxide stably, unaffected by the passage of time, also in a case where a strong acid is used as the acidic substance.

[Table 3]

| Concentration (ppm) of effective chlorine dioxide | | | |
|---|---|---|---|
| Time (days) | 1 mass% hydrochloric acid | 1 mass% sulfuric acid | 1 mass% citric acid |
| 0 | 465 | 495 | 485 |
| 0.125 | 450 | 445 | 455 |
| 1 | 365 | 360 | 425 |
| 3 | 375 | 280 | 380 |
| 9 | 280 | 240 | - |
| 28 | 220 | 170 | - |

(continued)

| Concentration (ppm) of effective chlorine dioxide | | | |
| --- | --- | --- | --- |
| Time (days) | 1 mass% hydrochloric acid | 1 mass% sulfuric acid | 1 mass% citric acid |
| 35 | 180 | 155 | 75 |

[Reference Signs List]

10: Double-structure container, 11: Pressure container (outer container), 12: Aerosol valve, 13A: First housing, 13B: Second housing, 14A: First stem, 14B: Second stem, 15A: First inner bag, 15B: Second inner bag, 16: Propellant filling space, 21: Actuator, 22A: First actuator passage, 22B: Second actuator passage, 23: Mixing space, 23A: Swirl passage, 24: Nozzle forming member, 24A: Discharge port
30: Aerosol product, 31a: First outer container, 31b: Second outer container, 32a: First inner container, 32b: Second inner container, 33a: Filling space for first propellant, 33b: Filling space for second propellant, 35: Actuator passage, 36: Actuator, 37: Fixed plate 1: Test stand, 2: Virus coated surface, 3: Petri dish

**Claims**

1. An aerosol product (10, 30) comprising: a first container (15a, 32a) filled with a first liquid; a second container (15b, 32b) filled with a second liquid; and a propellant, the aerosol product (10, 30) including a discharge mechanism (21) for discharging and mixing the first liquid and the second liquid,

   wherein the first liquid contains an aqueous solution of a chlorite salt,
   the second liquid contains an aqueous solution of an acidic substance,
   the acidic substance is citric acid,
   **characterized in that**
   the aqueous solution of the acidic substance has a concentration of 1 to 20 mass%, and
   the discharge mechanism (21) is configured so that the first liquid and the second liquid are mixed within the discharge mechanism (21) and thereafter discharged in mist form.

2. The aerosol product (10, 30) according to claim 1, wherein the chlorite salt includes sodium chlorite.

3. The aerosol product (10, 30) according to claim 1 or 2, wherein the aqueous solution of the chlorite salt has a pH of from 5 to 12.5.

4. The aerosol product (10, 30) according to any one of claims 1 to 3, wherein the aqueous solution of the chlorite salt has a concentration of 0.1 to 100,000 ppm, on a mass basis.

5. The aerosol product (10, 30) according to any one of claims 1 to 4, wherein the second liquid contains an additive.

6. The aerosol product (10) according to any one of claims 1 to 5, including an outer container (11) provided with a propellant filling space (16) filled with the propellant, the first container (15a), and the second container (15b), and the discharge mechanism (21) for discharging and mixing the first liquid and the second liquid from the first container (15a) and the second container (15b),
   wherein the propellant filling space (16) is formed between the first container (15a), the second container (15b) and the outer container (11).

7. The aerosol product (30) according to any one of claims 1 to 5, including a first inner container (32a) filled with the first liquid and a second inner container (32b) filled with the second liquid, a first outer container (31a) housing the first inner container (32a) and a second outer container (31b) housing the second inner container (32b), and the discharge mechanism (21) for discharging and mixing the first liquid and the second liquid,
   wherein spaces (33a, 33b) formed between the first inner container (32a) and the first outer container (3 1a), and between the second inner container (32b) and the second outer container (31b) are filled with a propellant.

**Patentansprüche**

1. Aerosolprodukt (10, 30), das Folgendes umfasst: einen ersten Behälter (15a, 32a), der mit einer ersten Flüssigkeit gefüllt ist; einen zweiten Behälter (15b, 32b), der mit einer zweiten Flüssigkeit gefüllt ist; und ein Treibmittel, wobei das Aerosolprodukt (10, 30) einen Ausstoßmechanismus (21) zum Ausstoßen und Mischen der ersten Flüssigkeit und der zweiten Flüssigkeit umfasst,

   wobei die erste Flüssigkeit eine wässrige Lösung eines Chloritsalzes enthält,
   die zweite Flüssigkeit eine wässrige Lösung einer sauren Substanz enthält,
   die saure Substanz Zitronensäure ist,
   **dadurch gekennzeichnet, dass**
   die wässrige Lösung der sauren Substanz eine Konzentration von 1 bis 20 Ma% aufweist, und
   der Ausstoßmechanismus (21) derart ausgestaltet ist, dass die erste Flüssigkeit und die zweite Flüssigkeit innerhalb des Ausstoßmechanismus (21) gemischt und danach in Nebelform ausgestoßen werden.

2. Aerosolprodukt (10, 30) nach Anspruch 1, wobei das Chloritsalz Natriumchlorit umfasst.

3. Aerosolprodukt (10, 30) nach Anspruch 1 oder 2, wobei die wässrige Lösung des Chloritsalzes einen pH-Wert von 5 bis 12,5 aufweist.

4. Aerosolprodukt (10, 30) nach einem der Ansprüche 1 bis 3, wobei die wässrige Lösung des Chloritsalzes bezogen auf die Masse eine Konzentration von 0,1 bis 100,000 ppm aufweist.

5. Aerosolprodukt (10, 30) nach einem der Ansprüche 1 bis 4, wobei die zweite Flüssigkeit einen Zusatzstoff enthält.

6. Aerosolprodukt (10) nach einem der Ansprüche 1 bis 5, das einen äußeren Behälter (11), der mit einem Treibmittelfüllraum (16), der mit dem Treibmittel gefüllt ist, dem ersten Behälter (15a) und dem zweiten Behälter (15b) versehen ist, und den Ausstoßmechanismus (21) zum Ausstoßen und Mischen der ersten Flüssigkeit und der zweiten Flüssigkeit von dem ersten Behälter (15a) und dem zweiten Behälter (15b) umfasst,
   wobei der Treibmittelfüllraum (16) zwischen dem ersten Behälter (15a), dem zweiten Behälter (15b) und dem äußeren Behälter (11) gebildet ist.

7. Aerosolprodukt (30) nach einem der Ansprüche 1 bis 5, das einen ersten inneren Behälter (32a), der mit der ersten Flüssigkeit gefüllt ist, und einen zweiten inneren Behälter (32b), der mit der zweiten Flüssigkeit gefüllt ist, einen ersten äußeren Behälter (31a), der den ersten inneren Behälter (32a) aufnimmt, und einen zweiten äußeren Behälter (31b), der den zweiten inneren Behälter (32b) aufnimmt, und den Ausstoßmechanismus (21) zum Ausstoßen und Mischen der ersten Flüssigkeit und der zweiten Flüssigkeit umfasst,
   wobei Räume (33a, 33b), die zwischen dem ersten inneren Behälter (32a) und dem ersten äußeren Behälter (31a) und zwischen dem zweiten inneren Behälter (32b) und dem zweiten äußeren Behälter (31b) gebildet sind, mit einem Treibmittel gefüllt sind.

**Revendications**

1. Produit aérosol (10, 30) comprenant : un premier récipient (15a, 32a) rempli d'un premier liquide ; un deuxième récipient (15b, 32b) rempli d'un deuxième liquide ; et un propulseur, le produit aérosol (10, 30) incluant un mécanisme de décharge (21) pour décharger et mélanger le premier liquide et le deuxième liquide,

   dans lequel le premier liquide contient une solution aqueuse d'un sel chlorite,
   le deuxième liquide contient une solution aqueuse d'une substance acide,
   la substance acide est l'acide citrique,
   **caractérisé en ce que**
   la solution aqueuse de la substance acide a une concentration de 1 à 20 % en masse, et
   le mécanisme de décharge (21) est configuré de façon que le premier liquide et le deuxième liquide soient mélangés à l'intérieur du mécanisme de décharge (21) et ensuite déchargés sous forme de brouillard.

2. Produit aérosol (10, 30) selon la revendication 1, dans lequel le sel chlorite inclut le chlorite de sodium.

3. Produit aérosol (10, 30) selon la revendication 1 ou 2, dans lequel la solution aqueuse du sel chlorite a un pH de 5 à 12,5.

4. Produit aérosol (10, 30) selon l'une quelconque des revendications 1 à 3, dans lequel la solution aqueuse du sel chlorite a une concentration, sur une base en masse, de 0,1 à 100 000 ppm.

5. Produit aérosol (10, 30) selon l'une quelconque des revendications 1 à 4, dans lequel le deuxième liquide contient un additif.

6. Produit aérosol (10, 30) selon l'une quelconque des revendications 1 à 5, incluant un récipient extérieur (11) doté d'un espace de remplissage de propulseur (16) rempli du propulseur, le premier récipient (15a), et le deuxième récipient (15b), et le mécanisme de décharge (21) pour décharger et mélanger le premier liquide et le deuxième liquide provenant du premier récipient (15a) et du deuxième récipient (15b),
dans lequel l'espace de remplissage de propulseur (16) est formé entre le premier récipient (15a), le deuxième récipient (15b) et le récipient extérieur (11).

7. Produit aérosol (10, 30) selon l'une quelconque des revendications 1 à 5, incluant un premier récipient intérieur (32a) rempli du premier liquide et un deuxième récipient intérieur (32b) rempli du deuxième liquide, un premier récipient extérieur (31a) logeant le premier récipient intérieur (32a) et un deuxième récipient extérieur (31b) logeant le deuxième récipient intérieur (32b), et le mécanisme de décharge (21) pour décharger et mélanger le premier liquide et le deuxième liquide,
dans lequel des espaces (33a, 33b) formés entre le premier récipient intérieur (32a) et le premier récipient extérieur (31a), et entre le deuxième récipient intérieur (32b) et le deuxième récipient extérieur (31b), sont remplis d'un propulseur.

Fig. 1

Fig. 2

Fig. 3

spraying

Fig. 4

**EP 3 738 925 B1**

## REFERENCES CITED IN THE DESCRIPTION

### Patent documents cited in the description

- JP 3110724 B
- JP 2004337582 A
- US 2013202484 A1
- US 2005079123 A1
- US 8840847 B2
- EP 2886625 A1